# EUROPEAN PATENT APPLICATION

(11) **EP 3 909 971 A1**
(43) Date of publication of application: **17.11.2021**
(21) Application number: 20198730.2
(22) Date of filing: 28.09.2020
(51) Int. Cl.: C07K 14/235, A61K 39/02

(54) **WHOLE CELL LIVESTOCK VACCINE FOR RESPIRATORY DISEASES**

(71) Applicant: Institute of Life Sciences (ILS), Bhubaneswar 751023 (IN)
(72) Inventor: DAS, Subrata Kumar, Bhubaneswar (IN); BADHAI, Jhasketan, Bhubaneswar (IN)
(74) Representative: Wunderlich & Heim Patentanwälte Partnerschaftsgesellschaft mbB

(57) **Abstract**

*Bordetella* species are primarily isolated from animals and humans causing asymptomatic infection to lethal pneumonia. Genomic ANI value and genome-wide SNPs-based phylogenetic tree suggest a divergent evolution of strain HT200 from a human-adapted lineage of *B. bronchiseptica.* Genome sequence revealed genes/ loci encoding the major *Bordetella* virulence factors, such as the dermonecrotic toxin (DNT), adenylate cyclase-hemolysin toxin (ACT), and lipopolysaccharide (LPS) O antigen are absent in strain HT200, while genes encoding other virulence factors, particularly pertactin (PRN), are highly divergent. *Bordetella bronchiseptica* strain HT200 does not produce the virulence factors - pertussis toxin (PTX) and serotype 2 fimbriae (FIM2). Additionally, strain HT200 was non-hemolytic, but showed hemagglutination activity and was able to colonize in the respiratory organs of mice and induced protective antibody response against challenge infection with pathogenic *B. bronchiseptica* strain RB50.

## Description

### FIELD OF THE INVENTION

The present invention relates to a vaccine for livestock, especially against respiratory diseases, and the process of preparation thereof.

### BACKGROUND AND PRIOR ARTS OF THE PRESENT INVENTION

Both live attenuated whole-cell and sub-unit (acellular) vaccines are commercially available to protect against *B. bronchiseptica* infections (1, 2, 3). However, the live attenuated *Bordetella bronchiseptica* vaccines have questionable safety (reversion of pathogenicity) and efficacy in animals (poor induction of immune response) (3). None of the commercial vaccine strains are genetically modified, besides the basis of their virulence attenuation are unknown, there is potential that the strains may revert to full or partial virulence (4). Moreover, currently available live attenuated whole-cell vaccines are not effective for booster vaccination of animals previously exposed to or immunized against *Bordetella bronchiseptica.* In this regard, *Bordetella bronchiseptica* strain HT200 that we proposed as a vaccine candidate is naturally attenuated and do not have the major virulence genes, such as dermonecrotic toxin (DNT) and adenylate cyclase-hemolysin (ACT). Moreover, the pertussis toxin (PTX) do not express by this bacterium, as a result of mutation in the promoter region. Furthermore, this strain can be used as a model organism for heterologous expression of pertussis toxin genes from *Bordetella pertussis* to enhance the efficacy of whole-cell vaccine.

### References:

1. John A. Ellis, G. Steven Krakowka, Arthur D. Dayton, Carrie Konoby. (2002). Comparative efficacy of an injectable vaccine and an intranasal vaccine in stimulating Bordetella bronchiseptica-reactive antibody responses in seropositive dogs.
2. John A. Ellis. (2015). How well do vaccines for Bordetella bronchiseptica work in dogs? A critical review of the literature 1977-2014. The Veterinary Journal 204: 5-16
3. D. Bottero, M. E. Zurita, M. E. Gaillard, E. Bartel, C. Vercellini, D. Hozbor. (2018). Membrane vesicles derived from Bordetella bronchiseptica: active constituent of a new vaccine against infections caused by this pathogen. Applied and Environmental Microbiology 84 (4): e01877-17.
4. Andrew Stevenson, Mark Roberts. (2003). Use of Bordetella bronchiseptica and Bordetella pertussis as live vaccines and vectors for heterologous antigens. FEMSImmunology andMedical Microbiology 37: 121-128.
5. Park J, Zhang Y, Buboltz AM, Zhang X, Schuster SC, Ahuja U, Liu M, Miller JF, Sebaihia M, Bentley SD, Parkhill J, Harvill ET. 2012. Comparative genomics of the classical Bordetella subspecies: the evolution and exchange of virulence-associated diversity amongst closely related pathogens. BMC Genomics 13: 545.
6. Buboltz AM, Nicholson TL, Parette MR, Hester SE, Parkhill J, Harvill ET. 2008. Replacement of adenylate cyclase toxin in a lineage of Bordetella bronchiseptica. J Bacteriol 190: 5502-5511.
7. Parkhill J, Sebaihia M, Preston A, Murphy LD, Thomson N, Harris DE, Holden MT, Churcher CM, Bentley SD, Mungall KL, Cerdeño-Tárraga AM, Temple L, James K, Harris B, Quail MA, Achtman M, Atkin R, Baker S, Basham D, Bason N, Cherevach I, Chillingworth T, Collins M, Cronin A, Davis P, Doggett J, Feltwell T, Goble A, Hamlin N, Hauser H, Holroyd S, Jagels K, Leather S, Moule S, Norberczak H, O'Neil S, Ormond D, Price C, Rabbinowitsch E, Rutter S, Sanders M, Saunders D, Seeger K, Sharp S, Simmonds M, Skelton J, Squares R, Squares S, Stevens K, Unwin L, Whitehead S, Barrell BG, Maskell DJ. 2003. Comparative analysis of the genome sequences of Bordetella pertussis, Bordetella parapertussis and Bordetella bronchiseptica. Nat Genet 35: 32-40.
8. Preston A, Parkhill J, Maskell DJ. 2004. The bordetellae: lessons from genomics. Nat Rev Microbiol 2: 379-390.

CN 101400783 provides a package Laid *coli* mutant strain, containing at least PTX gene mutation, deletion or mutated dnt, ampG gene and a heterologous gene. The package Laid Attenuated *Listeria* mutant strain may be used to treat or prevent bacterial infection packet Vater's immunogenic composition or vaccine. The package also provides a *Listeria* attenuated mutant strain Laid for the preparation of a vaccine or immunogenic composition, and a method of protecting a mammal package Laid *coli* resistance to infection.

AU 2012331646 discloses compositions, vaccines, and methods that include use of a mutated Bordetella strain against allergic diseases such as asthma and skin inflammation. This application further described kits, other compositions, vaccines, and methods.

US 9528086 discloses a genetically attenuated *Bordetella pertussis* strain including a mutated pertussis toxin (ptx) gene, and a heterologous ampG gene, and a hybrid protein including the N-terminal fragment of filamentous haemagglutinin (FHA) and a heterologous epitope or antigenic protein or protein fragment, different from FHA. The strain can be used in an attenuated vaccine for the treatment or prophylaxis of an infectious disease.

CA 2759280 relates to a life attenuated *Bordetella pertussis* vaccine which is deficient for tracheal cytotoxin (TCT), pertussis toxin (PTX), and dermonecrotic toxin (DNT) for prophylaxis or treatment of an allergen-driven airway pathology.

US 20080254062 pertains to *Bordetella* bacteria having a double mutation, a first mutation in a gene of the Type III secretion system and a second mutation in a gene of the adenylate cyclase toxin (CyaA) locus of the bacteria so that the mutations result in no Type III secretion system, a non-functional Type III secretion system, no CyaA protein, or a non-functional CyaA protein or a combination thereof. The *Bordetella* bacteria double mutant is attenuated while maintaining the efficacy of the bacteria to elicit an immune response. The present invention also pertains to vaccine compositions and methods for treating and immunizing a mammal against a disease caused by infection of *Bordetella* bacteria or a disease caused by a pathogen.

KR 101125244 relates to porcine reproductive and respiratory syndrome virus (Porcine Reproductive Respiratory Syndrome Virus, PRRSV) protein 5 (glycoprotein 5, GP5). The attenuated *Bordetella* chevron **kisep urticate** *(Bordetella bronchiseptica)* expressing in two kinds of antigen protein per antigen of the strain relates to a method for the prevention or treatment of infectious PRRSV using a disclosed method.

IN 2008DN07572 discloses a mutated *Bordetella* strain comprising at least a mutated ptx gene, a deleted or mutated dnt gene and a heterologous ampG gene. The attenuated mutated *Bordetella* strain can be used in an immunogenic composition or a vaccine for the treatment or prevention of a *Bordetella* infection. Further, this application claims use of the attenuated Bordetella strain for the manufacture of a vaccine or immunogenic composition, as well as methods for protecting mammals against infection by *Bordetella.*

AU 2017203244 relates to compositions, combinations, and methods comprising *Bordetella bronchiseptica* and isolated pertactin, which are effective in treating or 5 preventing respiratory infections, such as kennel cough, in animals.

CN 103261214 relates to a live or mutant bacteria, adjuvants, aromatic additives, or both aromatic additives and adjuvants suitable for systemic administration of the vaccine. Also, it relates to the vaccine by producing and method of administering the vaccine to protect the subject.

CN 107029230 provides a vaccine composition comprising a dog parainfluenza virus antigen with the immunity effective dose, a dog *Bordetella bronchiseptica* antigen with the immunity effective dose, and an adjuvant, wherein the dog parainfluenza virus antigen is selected from an inactivated dog parainfluenza virus antigen or at least one of F protein, HN protein, M protein, NP protein, P protein and L protein, and the *Bordetella bronchiseptica* antigen is selected from inactivated dog *Bordetella bronchiseptica* antigen or p68 protein. Compared with the vaccines in the prior art, the vaccine composition provided by the invention has the better immune effect, and has no side effects, and thus the risk that a veterinarian worker is infected is reduced. The invention further relates to a kit containing the inactivated dog parainfluenza virus antigen or the subunit antigen, the attenuated live dog *Bordetella bronchiseptica* antigen or the attenuated live dog parainfluenza virus antigen, and the inactivated dog *Bordetella bronchiseptica* antigen or p68 protein.

US 20050089533 relates to vaccines and methods for protecting dogs against disease caused by *Bordetella bronchiseptica.* This invention relates to vaccines and methods for protecting dogs against disease caused by *Leptospira bratislava.* This invention also relates to combination vaccines and methods for protecting dogs against disease or disorder caused by canine pathogens, for example, infectious tracheobronchitis caused by *Bordetella bronchiseptica,* canine distemper caused by canine distemper (CD) virus, infectious canine hepatitis (ICH) caused by canine adenovirus type 1 (CAV-1), respiratory disease caused by canine adenovirus type 2 (CAV-2), canine parainfluenza caused by canine parainfluenza (CPI) virus, enteritis caused by canine coronavirus (CCV) and canine parvovirus (CPV), and leptospirosis caused by *Leptospira bratislava, Leptospira canicola, Leptospira grippotyphosa, Leptospira icterohaemorrhagiae* or *Leptospira pomona.*

### OBJECTS OF THE PRESENT INVENTION:

It is therefore an object of this invention to design a live attenuated vaccine candidates or immunogenic composition against general respiratory problems of an individual, especially an animal, more specifically domestic animals.

Another object of the present invention is to provide a vaccine or immunogenic composition which induces protection in new-borns as well as in all animals of any age.

Yet another object of the present invention to provide a mucosal and systemic immunity inducing vaccine or immunogenic composition.

Still another object of the present invention is to provide a live attenuated *Bordetella bronchiseptica* HT200 having altered virulence factors, and colonizing ability in the respiratory tracts.

Further object of the present invention is to disclose the use of a vaccine comprising live attenuated *Bordetella bronchiseptica* HT200.

Still further object of this invention is to provide a method of protective treatment against respiratory diseases in general, including but not limited to *Bordetella bronchiseptica* RB50.

These and other objects and advantages of the invention will be apparent from the ensuing description.

### SUMMARY OF THE INVENTION

In one aspect of the invention, the present invention discloses and claims a live attenuated *Bordetella bronchiseptica* HT200 strain with sequence divergence amounts to < 98.5 % nucleotide sequence identity, lacking dermonecrotic toxin (*dnt*) gene and gene for lipopolysaccharide O antigen, *cya* operon responsible for the secretion of adenylate cyclase hemolysin (*act*) replaced by *the ptp* operon with sequence divergence at other known *Bordetella* virulence genes and loci including the pertussis toxin production locus (*ptxABCDE*)*,* filamentous hemagglutinin and fimbriae biogenesis loci (*fhaB-fimABCD-fhaC*)*,* fimbrial major subunit genes (*fimN, fimX, fim2,* and *fim3),* other filamentous hemagglutinins-like genes (*fhaL,* and *fhaS*), tracheal colonization factor (*tcfA*)*,* autotransporter pertactin gene (*prn*)*, Bordetella* resistance to killing genes (*brkAB*)*,* type three secretion system (T3SS) and regulatory loci (*bsc-btr*)*,* T3SS effector genes (*bteA, bopB, bopD, bopN,* and *bsp22*)*,* iron acquisition system loci (alcaligin, *alcABCDERS-fauA;* enterobactin, *bfeRAB;* and heme, *hurIR-bhuRSTUV*)*,* flagellin (*flaA*)*,* and the *Bordetella* virulence gene regulatory locus (*bvgASR*)*,* wherein said strain showed an indistinctive or cryptic expression of the virulence factors - FHA (filamentous hemagglutinin) and FIM3 (serotype 3 fimbriae) but no expression of PTX-S1 (pertussis toxin subunit 1) and FIM2 (serotype 2 fimbriae). The strain expresses hemagglutination activity, able to colonize in the respiratory organs inducing protective antibody response against infection of virulent *Bordetella bronchiseptica* RB50 strain.

In another aspect of the invention, the present invention discloses a method for enhancing the immune response toward a microbial pathogen including but not limited to influenza virus, Respiratory Syncytial Virus (SRV), against *Streptococcus pneumoniae,* infection of virulent *Bordetella bronchiseptica* RB50 strain in a subject, said method comprising administering to a subject the live attenuated *Bordetella bronchiseptica* HT200 strain comprising a mutated pertussis toxin (*ptx*) gene, lacking dermonecrotic toxin (*dnt*) gene, *cya* operon responsible for the secretion of adenylate cyclase hemolysin (*act*) replaced by the *ptp* operon, indistinctive or cryptic expression of the virulence factors - FHA (filamentous hemagglutinin) and FIM3 (serotype 3 fimbriae) but no expression of PTX-S1 (pertussis toxin subunit 1) and FIM2 (serotype 2 fimbriae).

In yet another aspect of the invention, the present invention discloses a method of reducing especially a T-lymphocyte helper type 1 (Th-1)-dominated allergic reaction in a subject against infection of *Bordetella bronchiseptica* RB50 strain, *Streptococcus pneumoniae,* Respiratory Syncytial Virus (SRV), influenza virus, said method comprises colonizing the respiratory tract of the sample subject prior the onset of an allergic reaction with a live attenuated *Bordetella bronchiseptica* HT200 strain, wherein the strain having altered production of functional pertussis toxin, dermonecrotic toxin, and tracheal cytotoxin while retaining the colonizing ability, wherein the colonization of the mammal elicits an anti-inflammatory response which reduces the Th1-dominated allergic reaction in said subject, wherein live attenuated *Bordetella bronchiseptica* HT200 strain comprising a mutated pertussis toxin (*ptx*) gene due to mutation in the promoter region of said gene, deleted dermonecrotic toxin (*dnt*) gene, deleted gene for lipopolysaccharide O antigen, *ptp* operon replacing *cya* operon responsible for the secretion of adenylate cyclase hemolysin (*act*).

In still another aspect of the present invention, it discloses a vaccine to be administered through routes including but not limited to nasal route comprising live attenuated *Bordetella bronchiseptica* HT200 strain with sequence divergence amounts to < 98.5 % nucleotide sequence identity, comprising essentially of a mutated pertussis toxin (*ptx*) gene due to at least one mutation in the promoter region of said gene leading to an enzymatically inactive protein that may retain immunogenic properties, deleted dermonecrotic toxin (*dnt*) gene, deleted gene for lipopolysaccharide O antigen, *ptp* operon replacing *cya* operon responsible for the secretion of adenylate cyclase hemolysin (*act*), with sequence divergence at other known *Bordetella* virulence genes and loci including the pertussis toxin production locus (*ptxABCDE*), filamentous hemagglutinin and fimbriae biogenesis loci (*fhaB-fimABCD-fhaC*)*,* fimbrial major subunit genes (*fimN, fimX, fim2,* and *fim3),* other filamentous hemagglutinins-like genes (*fhaL,* and *fhaS*), tracheal colonization factor (*tcfA*)*,* autotransporter pertactin gene (*prn*)*, Bordetella* resistance to killing genes (*brkAB*)*,* type three secretion system (T3SS) and regulatory loci (*bsc-btr*)*,* T3SS effector genes (*bteA, bopB, bopD, bopN,* and *bsp22),* iron acquisition system loci (alcaligin, *alcABCDERS-fauA;* enterobactin, *bfeRAB;* and heme, *hurIR-bhuRSTUV*)*,* flagellin (*flaA*)*,* and the *Bordetella* virulence gene regulatory locus (*bvgASR*)*,* wherein said strain showed an indistinctive or cryptic expression of the virulence factors - FHA (filamentous hemagglutinin) and FIM3 (serotype 3 fimbriae) but no expression of PTX-S1 (pertussis toxin subunit 1) and FIM2 (serotype 2 fimbriae).

In further aspect, the present invention discloses a use of a live attenuated *Bordetella bronchiseptica* HT200 strain for the manufacture of a medicament for reducing a T-lymphocyte helper type 1 (Th-1)-dominated allergic reaction during pneumonia in a subject, wherein the strain is mutated to reduce its production of functional pertussis toxin, dermonecrotic toxin, and tracheal cytotoxin while retaining the ability to colonize the mammal, and wherein colonization of the mammal with the strain elicits an anti-inflammatory response which reduces the Th1-dominated allergic reaction in the mammal, wherein colonization of the mammal with the strain occurs prior to onset of the allergic reaction in the respiratory tracts of the subject.

### BRIEF DESCRIPTION OF ACCOMPANYING DRAWINGS

The illustrated embodiments of the subject matter will be best understood by reference to the drawings, wherein like parts are designated by like numerals throughout. The following description is intended only by way of example., and simply illustrates certain selected embodiments of devices, systems, and processes that are consistent with the subject matter as claimed herein, wherein:

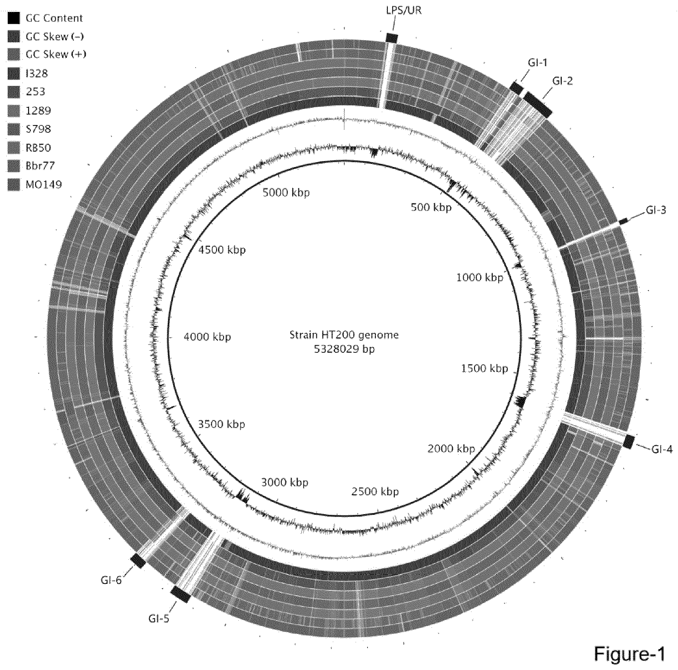

- **Figure 1:**: Genomic comparison of *B. bronchiseptica* HT200 with reference strains. From inside to outside: ring 1, genome of strain HT200; ring 2, GC content; ring 3, GC skewness; rings 4 to 11, reference genome of strain 1328, 253, 1289, S798, RB50, Bbr77, and M0149, respectively; ring 12, accessory large genomic regions in strain HT200: LPS/UR, (size, 32.3 kb; GC mol %, 58.9; CDSs, 24); GI-1, (size, 33.9 kb; GC mol %, 59.9; CDSs, 28); GI-2, (size, 68.2; GC mol %, 65.1; CDSs, 68); GI-3, (size, 12.6 kb; GC mol %, 58.7; CDSs, 7); GI-4, (size, 36.7 kb; GC mol %, 56.2; CDSs, 22); GI-5, (size, 55.6 kb; GC mol %, 60.3; CDSs, 43); and GI-6, (size, 40.2 kb; GC mol %, 65.4; CDSs, 56). LPS/UR, Lipopolysaccharide/Unique Region; GI, Genomic Island; kb, kilo base; CDS, Coding DNA sequence.

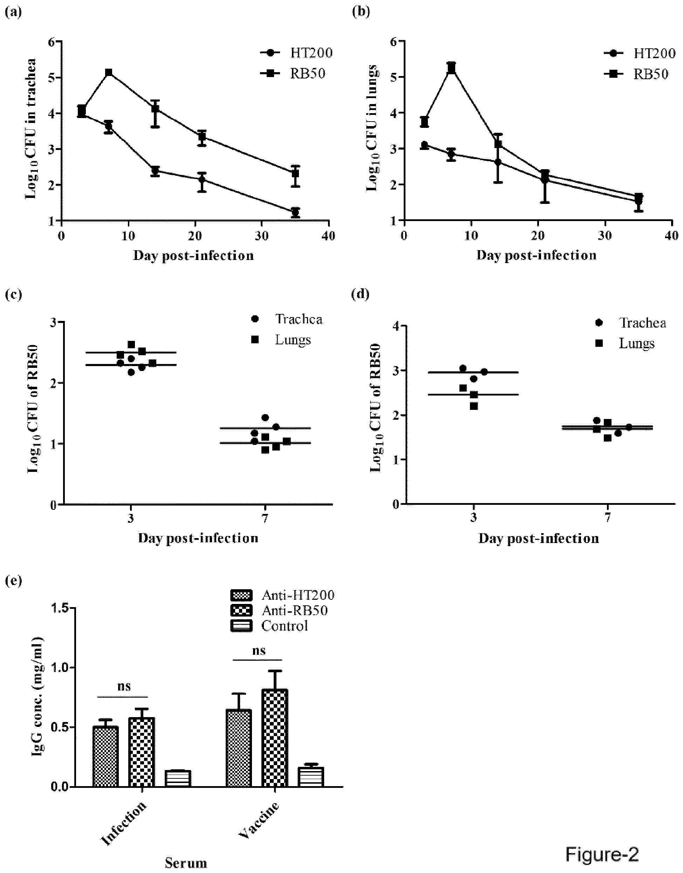

- **Figure 2:**: Bacterial colonization in mice respiratory organs: **(a)** trachea, and **(b)** lungs. 4-6 weeks old C57BL/6 mice, in groups of 3-4, were intranasally inoculated with bacteria by pipetting 50 µl of the inoculum (5 × 10⁶ CFU) in PBS onto the tip of the external nares. **(c)** Infection-induced clearance of *B. bronchiseptica* strain RB50. Group of 4 C57BL/6 mice were challenged with *B. bronchiseptica* strain RB50 (5 × 10⁷ CFU) 40 days after initial infection with B. *bronchiseptica* strain HT200 (5 × 10⁶ CFU). (d) Vaccine-induced clearance of *B. bronchiseptica* strain RB50. Group of 3 C57BL/6 mice were challenged with *B. bronchiseptica* strain RB50 (5 × 10⁷ CFU) at 28 days' post-vaccination with heat-killed whole-cells of strain HT200 (10⁸ CFU). (e) Total serum IgG antibody titers. Sera were collected from groups of 3-4 C57BL/6 mice after 28 days of infection with 5 × 10⁶ CFU and subcutaneous injection of 10⁸ CFU of heat-killed whole cells of strains HT200 and RB50, respectively, and the antibody titers were estimated by ELISA.

### DETAILED DESCRIPTION OF THE INVENTION

At the very outset of the detailed description, it may be understood that the ensuing description only illustrates a form of this invention. However, such a form is only exemplary embodiment, and without intending to imply any limitation on the scope of this invention. Accordingly, the description is to be understood as an exemplary embodiment and teaching of invention and not intended to be taken restrictively.

Throughout the description and claims of this specification, the phrases "comprise" and "contain" and variations of them mean "including but not limited to", and are not intended to exclude other moieties, additives, components, integers or steps. Thus, the singular encompasses the plural unless the context otherwise requires. Wherever there is an indefinite article used, the specification is to be understood as contemplating plurality as well as singularity, unless the context requires otherwise.

Thus, the terms "comprises", "comprising", or any other variations thereof used in the disclosure, are intended to cover a non-exclusive inclusion, such that a device, system, assembly that comprises a list of components does not include- only those components but may include other components not expressly listed or inherent to such system, or assembly, or device.

In other words, one or more elements in a system or device proceeded by "comprises... a" does not, without more constraints, preclude the existence of other elements or additional elements in the system, apparatus or device. Features, integers, characteristics, compounds, chemical moieties or groups described in conjunction with an aspect, embodiment or example of the invention are to be understood to be applicable to any other aspect, embodiment or example described herein unless incompatible therewith. All the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification including any accompanying claims, abstract and drawings or any parts thereof, or to any novel one, or any novel combination, of the steps of any method or process so disclosed. The reader's attention is directed to all papers and documents which are filed concurrently with or before this specification in connection with this application and which are open to public inspection with this specification, and the contents of all such papers and documents are incorporated herein by reference. Post filing patents, original peer reviewed research paper shall be published.

The following descriptions of embodiments and examples are offered by way of illustration and not by way of limitation.

Unless contraindicated or noted otherwise, throughout this specification, the terms "a" and "an" mean one or more, and the term "or" means and/or. As used in the description herein and throughout the claims that follow, the meaning of "a." "an," and "the" includes plural reference unless the context clearly dictates otherwise. Also, as used in the description herein, the meaning of "in" includes "in" and "on" unless the context clearly dictates otherwise.

It may especially be noted that no animals have been harmed intentionally or unintentionally for the purposes of this invention. The animals used for the purpose of confirming the safety and efficacy of the present invention, were all treated with utmost care, and the smallest possible wound is made in a controller manner following the treatment of the same with the outcome of the present invention.

Thus, according to this invention is provided a *Bordetella* species, primarily isolated from animals and humans causing asymptomatic infection to lethal pneumonia. Genome sequence revealed genes encoding the major *Bordetella* virulence factors - DNT and ACT are absent in strain HT200, while genes encoding other virulence factors are highly divergent. Strain HT200 does not express the virulence factors - pertussis toxin and serotype 2 fimbriae. Additionally, strain HT200 was non-hemolytic, but showed hemagglutination activity and was able to colonize in the respiratory organs of mice and induced protective antibody response against challenge infection with pathogenic B. *bronchiseptica* strain RB50. Results obtained from mice infection demonstrated that *B. bronchiseptica* strain HT200 is a naturally attenuated bacterium and could be a safer candidate for development of live or heat killed whole-cell vaccine for immunization against pathogenic *B. bronchiseptica* infection.

*Bordetella bronchiseptica* strain HT200 (a Gram-negative bacterium isolated from thermal spring).

Thus, according to another aspect of the same invention, there is provided use of the vaccine in specific cases, a method of protecting from respiratory diseases, preferably before the onset of an infection.

The present invention as accordance to the subject matter of concern, Strain HT200 is aerobic, Gram-negative, motile, coccobacillus (0.2-0.7 × 0.5-1.5 µm), and formed smooth colonies on TSA/BGA plates. Growth occurred at 20 - 40°C temperature and the optimum growth was observed at 35 - 37 °C. 16S rRNA gene sequence analysis showed strain HT200 belonged to the genus *Bordetella* and clustered with the three closely related host-restricted 'classical' mammalian species (1-4); and have similarity with *B. parapertussis* (99.52 %), *B. bronchiseptica* (99.39 %), and *B. pertussis* (99.25 %). Strain HT200 was positive for activities of catalase, oxidase, urease, and nitrate reduction reactions; these phenotypes are similar with the *B. bronchiseptica.*

The genome of strain HT200 was 5.3 Mb. The final genome assembly consisted of a single chromosome with a GC content of 68.16 mol %. The genome comprised of 5061 genes, 4510 coding sequences (CDSs), 55 tRNAs, 9 rRNAs (3, 5S rRNA; 3, 16S rRNA and 3, 23S rRNA), 4 non-coding (nc) RNAs, and 1 clustered regularly interspaced short palindromic repeat (CRISPR).

Pairwise genome comparison revealed the genome of strain HT200 has high degree of synteny conservation with the genomes of the human isolates 1328 (size, 5.08 Mb) and MO149 (size, 5.09 Mb) than with the genome of the rabbit isolate RB50 (size, 5.34 Mb). Further, horizontal gene transfer (HGT) analysis predicted acquisition of six large (> 10 kb size) genomic islands (GIs), based on their differential DNA GC-content and -skewness, at discrete locations in the genome of strain HT200 (Fig. 1).

Genome of strain HT200 contains homolog of most of the *Bordetella* virulence protein-encoding genes and loci but lacks *dnt* encoding dermonecrotic toxin (DNT) and the *cya* responsible for production and secretion of adenylate cyclase-hemolysin toxin (ACT). Moreover, the *cya* operon was replaced by a *ptp* operon encoding predicted peptide transport proteins. Earlier studies have shown that in many *B. bronchiseptica* isolates of human origin, the *dnt* gene is either missing *(B. bronchiseptica* strain Bbr77, *B. bronchiseptica* strain D445 or divergent (as low as 93 % identity in *B. bronchiseptica* strain MO140) compared to the isolates from animal hosts (1). Similarly, Buboltz and colleagues (2) have reported the replacement of *cya* operon by a *ptp* operon in *B. bronchiseptica* strains 253, a hypo virulent dog isolate and other strains of the same phylogenetic lineage. Additionally, the entire *wbm* genetic locus and part of *wlb* locus responsible for the LPS O-antigen and band A (outer core trisaccharide) biosynthesis, respectively in *B. bronchiseptica* strain RB50 (2, 22) are missing in strain HT200. Moreover, strain HT200 exhibited sequence divergence (< 98.5 % nucleotide sequence identity) at other known *Bordetella* virulence genes and loci including the pertussis toxin production locus (*ptxABCDE*), filamentous hemagglutinin and fimbriae biogenesis loci (*JhaB-fimABCD-fhaC*)*,* fimbrial major subunit genes (*fimN, fimX, fim2,* and *fim3),* other filamentous hemagglutinins-like genes (*fhaL,* and *fhaS*), tracheal colonization factor (*tcfA*)*,* autotransporter pertactin gene (*prn*)*, Bordetella* resistance to killing genes (*brkAB*)*,* type three secretion system (T3SS) and regulatory loci (*bsc-btr*)*,* T3SS effector genes *(bteA, bopB, bopD, bopN,* and *bsp22),* iron acquisition system loci (alcaligin, *alcABCDERS-fauA;* enterobactin, *bfeRAB;* and heme, *hurIR-bhuRSTUV*)*,* flagellin (*flaA*)*,* and the *Bordetella* virulence gene regulatory locus (*bvgASR*) (Fig. S3). The highest nucleotide sequence variation was found in the *prn* gene encoding the pertactin protein (69 % identity). Multiple sequence alignment of the translated pertactin (PRN) protein showed most of the variations were in the N-terminal amino acid sequences in strains HT200 and 1328. Further, genome of strain HT200 contains an intact putative capsule locus (CUR95_20305 to CUR95_20385) that has been suggested to contribute to persistence of *B. bronchiseptica* in the environment, instead of being involved in pathogenesis in the mammalian host (3, 4). Consistent with their shared phylogeny, *B. bronchiseptica* strain 1328 showed conservation in the nucleotide sequences (> 99% identity) as well as presence or absence of virulence genes and loci. Clearly, comparative genomics data suggests that the strain HT200 and strain 1328, have evolved from a lineage of *B. bronchiseptica* ancestor distinct from the lineage of other classical Bordetellae. Phenotypically strain HT200 was non-haemolytic on sheep blood agar plates but tested positive for hemagglutination activity using mammalian erythrocytes (guinea pig and rabbit). Besides, strain HT200 showed an indistinctive or cryptic expression of the virulence factors - FHA (filamentous hemagglutinin) and FIM3 (serotype 3 fimbriae) but no expression of PTX-S1 (pertussis toxin subunit 1) and FIM2 (serotype 2 fimbriae).

*B. bronchiseptica* is an important veterinary pathogen which has been isolated from a variety of mammalian host including humans. The genome of strain HT200 contained homologs of many virulence genes that are commonly found in *Bordetella* species pathogenic to *humans* and other animals. Results obtained from mice infection demonstrated that *B. bronchiseptica* strain HT200 is a naturally attenuated bacterium and could be a safer candidate for development of live or heat killed whole-cell vaccine for immunization against pathogenic B. *bronchiseptica* infection.

*Bordetella* species are primarily isolated from animals and humans causing asymptomatic infection to lethal pneumonia. Genome sequence revealed genes/loci encoding the major *Bordetella* virulence factors, such as the dermonecrotic toxin (DNT), adenylate cyclase-hemolysin toxin (ACT), and lipopolysaccharide (LPS) O antigen are absent in strain HT200, while genes encoding other virulence factors, particularly pertactin (PRN), are highly divergent. *Bordetella bronchiseptica* strain HT200 does not produce the virulence factors - pertussis toxin (PTX) and serotype 2 fimbriae (FIM2). Additionally, B. *bronchiseptica* strain HT200 is non-hemolytic, but showed hemagglutination activity *in vitro* and was able to colonize in the respiratory organs of C57BL/6 mice and induced protective IgG antibody response against challenge infection with pathogenic *B. bronchiseptica* strain RB50. Results obtained from mice infection demonstrated that *B. bronchiseptica* strain HT200 is a naturally attenuated bacterium and could be a safer candidate for development of live or heat killed whole-cell vaccine for immunization against pathogenic B. *bronchiseptica* infection.

In one aspect of the invention, the present invention discloses and claims a live attenuated *Bordetella bronchiseptica* HT200 strain with sequence divergence amounts to < 98.5 % nucleotide sequence identity, comprising a mutated pertussis toxin (*ptx*) gene due to mutation in the promoter region of said gene leading to an enzymatically inactive protein that may retain immunogenic properties, deleted dermonecrotic toxin (*dnt*) gene, deleted gene for lipopolysaccharide O antigen, *ptp* operon replacing *cya* operon responsible for the secretion of adenylate cyclase hemolysin (*act*), with sequence divergence at other known *Bordetella* virulence genes and loci including the pertussis toxin production locus (*ptxABCDE*), filamentous hemagglutinin and fimbriae biogenesis loci (*fhaB-fimABCD-fhaC*), fimbrial major subunit genes (*fimN, fimX, fim2,* and *fim3*)*,* other filamentous hemagglutinins-like genes (*fhaL,* and *fhaS*), tracheal colonization factor (*tcfA*)*,* autotransporter pertactin gene (*prn*)*, Bordetella* resistance to killing genes (*brkAB*)*,* type three secretion system (T3SS) and regulatory loci (*bsc-btr*)*,* T3SS effector genes (*bteA, bopB, bopD, bopN,* and *bsp22*)*,* iron acquisition system loci (alcaligin, *alcABCDERS-fauA;* enterobactin, *bfeRAB;* and heme, *hurIR-bhuRSTUV*)*,* flagellin (*flaA*)*,* and the *Bordetella* virulence gene regulatory locus (*bvgASR*)*,* wherein said strain showed an indistinctive or cryptic expression of the virulence factors - FHA (filamentous hemagglutinin) and FIM3 (serotype 3 fimbriae) but no expression of PTX-S1 (pertussis toxin subunit 1) and FIM2 (serotype 2 fimbriae). The strain expresses hemagglutination activity, able to colonize in the respiratory organs inducing protective antibody response against infection of virulent *Bordetella bronchiseptica* RB50 strain.

In another aspect of the invention, the present invention discloses a method for enhancing the immune response toward a microbial pathogen including but not limited to influenza virus, Respiratory Syncytial Virus (SRV), against *Streptococcus pneumoniae,* infection of virulent *Bordetella bronchiseptica* RB50 strain in a subject, said method comprising administering to a subject the live attenuated *Bordetella bronchiseptica* HT200 strain comprising a mutated pertussis toxin *(ptx)* gene, lacking dermonecrotic toxin *(dnt)* gene, *cya* operon responsible for the secretion of adenylate cyclase hemolysin (*act*) replaced by the *ptp* operon, indistinctive or cryptic expression of the virulence factors - FHA (filamentous hemagglutinin) and FIM3 (serotype 3 fimbriae) but no expression of PTX-S1 (pertussis toxin subunit 1) and FIM2 (serotype 2 fimbriae), wherein the gene encoding the hybrid protein is inserted into the locus of the deleted *dnt* gene and the *Bordetella pertussis* strain lacks a native FHA protein, and wherein the heterologous antigen comprises an antigen from the microbial pathogen.

In still another aspect of the present invention, it discloses a vaccine to be administered through routes including but not limited to nasal route comprising live attenuated *Bordetella bronchiseptica* HT200 strain with sequence divergence amounts to < 98.5 % nucleotide sequence identity, comprising essentially of a mutated pertussis toxin *(ptx)* gene, lacking dermonecrotic toxin *(dnt)* gene, *cya* operon responsible for the secretion of adenylate cyclase hemolysin (*act*) replaced by the *ptp* operon, with sequence divergence at other known *Bordetella* virulence genes and loci including the pertussis toxin production locus (*ptxABCDE*), filamentous hemagglutinin and fimbriae biogenesis loci (*fhaB-fimABCD-fhaC*), fimbrial major subunit genes (*fimN, fimX, fim2,* and *fim3*)*,* other filamentous hemagglutinins-like genes (*fhaL,* and *fhaS*), tracheal colonization factor (*tcfA*)*,* autotransporter pertactin gene (*prn*)*, Bordetella* resistance to killing genes (*brkAB*)*,* type three secretion system (T3SS) and regulatory loci (*bsc-btr*)*,* T3SS effector genes (*bteA, bopB, bopD, bopN,* and *bsp22*), iron acquisition system loci (alcaligin, *alcABCDERS-fauA;* enterobactin, *bfeRAB;* and heme, *hurIR-bhuRSTUV*)*,* flagellin (*flaA*)*,* and the *Bordetella* virulence gene regulatory locus (*bvgASR*)*,* wherein said strain showed an indistinctive or cryptic expression of the virulence factors - FHA (filamentous hemagglutinin) and FIM3 (serotype 3 fimbriae) but no expression of PTX-S1 (pertussis toxin subunit 1) and FIM2 (serotype 2 fimbriae).

*Bordetella* colonies on Bordet-Gengou agar are known to exist in distinct morphological types. These have been classed into Phase I, II and III. See, Peppler et al., Infect. Immun. 44 (3):681 (1984). In this invention, live attenuated *Bordetella* are preferably employed.

For the purpose of initial growth and propagation, the *Bordetella* organisms can be cultured on a variety of nutrient media. Bordet-Gengou medium (BG) and synthetic minimal medium, may be used. Both can be prepared using commercially available media. BG, for example, can be prepared by adding 20% defibrinated sheep blood to basal medium such as is supplied by BBL. Strain B. *bronchiseptica* may be inoculated on a blood agar medium (TS blood agar medium) and cultured at a definite temperature. The grown cells are harvested in a phosphate buffered saline (PBS of pH 7.0) to give a cell suspension. To the cell suspension N-methyl-N'-nitro-N-nitrosoguanidine, may be added and the mixture is incubated with shaking at 32° C. for one hour. The culture solution is then irradiated with ultraviolet ray. The resulting solution is centrifuged and then is washed with the same phosphate buffered saline as above. The resulting cells are resuspended in the same phosphate buffered saline and are cultured on the same blood agar medium. From the resulting colonies, urease negative strain is collected in accordance with urease test.

*Bordetella* organisms are cultured at 30° to 45° C., at approximately neutral pH, e.g., 6.5-7.5, preferably 6.7-7.1. For best growth, liquid cultures are agitated and aerated throughout fermentation. To prepare production cultures, seed cultures are serially propagated in accordance with standard techniques. Production cultures are seeded with at least a 1%, preferably 5 to 10%, inoculum of actively growing cells. Fermentation, prior to inactivation, is typically continued until nutrients are substantially depleted, i.e., depleted or nearly depleted, e.g., 18 to 36 hours. The organisms used in bacterin preparation are passaged for propagation and preferably remain substantially non-attenuated throughout serial passage and production culture.

To prepare the bacteria used in vaccine formulation, glutaraldehyde may be added to an active culture having achieved a cell density a definite nephelometric units. Nephelometric units are determined using a NephoColorimeter which has been calibrated with a standard of certified accuracy, for example, a Coleman Certified Nephelo Standard N=78, and a dilution blank solution, for example, physiological saline. The method used to calibrate the Nephelo-Colorimeter is commonly referred to as the "null method" in which the instrument has been calibrated such that a linear relationship exists for all nephelo values between the dilution blank solution, which is zero, and the certified standard. Samples of unknown nephelometric values may be diluted stoichiometrically in dilution blank solution so that when measured in the calibrated instrument, this value is then multiplied by the dilution factor to determine the nephelometric value of the undiluted sample.

The strain is suspended in a phosphate buffered saline (pH 7.0), and the diluted cell suspension is cultured on a blood agar medium (BG medium). From a comparatively small colony produced after more than 3 days, a cell is picked up and is grown on the same medium as above. The culture thus obtained is cultured again and the cell is picked up in the same manner as above. After repeating ten times the above procedure, there is obtained phase I organism grown with a small colony which shows strong hemolytic property, via phase II organism having weak hemolytic property.

The present formulation is further clarified by giving the following exhibits. It must, however, be understood that these exhibits are only illustrative in nature and should not be taken as limitations to the capacity of the invention. Several amendments and improvements to the disclosed segments will be obvious to those skilled in the art. Thus, these amendments and improvements may be made without deviating from the scope of the invention.

### Example 1:

### Colonization and Induction of Protective Immune Response:

Colonization efficacy of strain HT200 in the respiratory organs (lung and trachea) was tested by infecting C57BL/6 mice. 4-6 weeks old C57BL/6 mice were intranasally inoculated with strain HT200 or RB50 and the colonization was estimated over a period of 35 days. Strain HT200 was able to colonize in the trachea and lungs in mice (Fig. 2a & 2b); however, the colony forming units (CFU) of strain HT200 was relatively lower compared to the reference strain RB50 (Fig. 2a). Examination of the trachea and lungs of mice did not reveal signs of any pathological damage. Moreover, strain HT200 induced protective immune response in C57BL/6 mice against challenge infection with lethal dose of the pathogenic strain RB50; both infection-induced and heat-killed whole-cell vaccine-induced antibody response provided protection to the mice (Fig. 2c & 2d). Measurement of the total reactive IgG antibody titres indicated the presence of equivalent amount of protective IgG antibodies against strain HT200 (environmental isolate) and strain RB50 (pathogenic rabbit isolate) in the mice sera (Fig. 2e).

### Example 2:

### Bacterial colonization in mice respiratory organs:

Respiratory organs namely (a) trachea, and (b) lungs, of 4-6 weeks old C57BL/6 mice, in groups of 3-4, were intranasally inoculated with bacteria by pipetting 50 µl of the inoculum (5 × 10⁶ CFU) in PBS onto the tip of the external nares. (c) Infection-induced clearance of *B. bronchiseptica* strain RB50. Group of 4 C57BL/6 mice were challenged with *B. bronchiseptica* strain RB50 (5 × 10⁷ CFU) 40 days after initial infection with *B. bronchiseptica* strain HT200 (5 × 10⁶ CFU). (d) Vaccine-induced clearance of *B. bronchiseptica* strain RB50. Group of 3 C57BL/6 mice were challenged with *B. bronchiseptica* strain RB50 (5 × 10⁷ CFU) at 28 days' post-vaccination with heat-killed whole-cells of strain HT200 (10⁸ CFU). (e) Total serum IgG antibody titers. Sera were collected from groups of 3-4 C57BL/6 mice after 28 days of infection with 5 × 10⁶ CFU and subcutaneous injection of 10⁸ CFU of heat-killed whole cells of strains HT200 and RB50, respectively, and the antibody titers were estimated by ELISA.

In sum, the present invention, although works with certain peptides, and designs certain DNA sequences, in no condition, these may be considered as "Biological Material" as per the definition of the Biodiversity Act, saying "Biological resources as plants, animals and micro-organisms or parts thereof, their genetic material and by-products (excluding value added products) with actual or potential use or value, but does not include human genetic material", therefore, this present invention may have nothing to do with the living world except for working with certain proteins, which are merely biochemical entities, easily synthesized in laboratory conditions. Neither the proteins are parts of genetic material, nor there is any genetic sequence in designed primers. All the source and geographical origins are, however, categorically disclosed.

Now, the crux of the invention is claimed implicitly and explicitly through the following claims.

Each of the appended claims defines a separate invention, which for infringement purposes is recognized as including equivalents to the various elements or limitations specified in the claims. Depending on the context, all references below to the "invention" may in some cases refer to certain specific embodiments only. In other cases, it will be recognized that references to the "invention" will refer to subject matter recited in one or more, but not necessarily all, of the claims.

Groupings of alternative elements or embodiments of the invention disclosed herein are not to be construed as limitations. Each group member can be referred to a claimed individually or in any combination with other members of the group or other elements found herein. One or more members of a group can be included in or deleted from, a group for reasons of convenience d/ or patentability. When any such inclusion or deletion occurs, the specification is herein deemed to contain the group as modified thus fulfilling the written description of all groups used in the appended claims.

## Claims

1. A live attenuated *Bordetella bronchiseptica* HT200 strain comprising a mutated pertussis toxin (*ptx*) gene due to mutation in the promoter region of said gene, deleted dermonecrotic toxin *(dnt)* gene, deleted gene for lipopolysaccharide O antigen, *ptp* operon replacing *cya* operon responsible for the secretion of adenylate cyclase hemolysin (*act*), with sequence divergence at other known *Bordetella* virulence genes and loci including the pertussis toxin production locus (*ptxABCDE*), filamentous hemagglutinin and fimbriae biogenesis loci (*fhaB-fimABCD-fhaC*)*,* fimbrial major subunit genes (*fimN, fimX, fim2,* and *fim3),* other filamentous hemagglutinins-like genes (*fhaL,* and *fhaS*)*,* tracheal colonization factor (*tcfA*)*,* autotransporter pertactin gene (*prn*)*, Bordetella* resistance to killing genes (*brkAB*)*,* type three secretion system (T3SS) and regulatory loci (*bsc-*b*tr),* T3SS effector genes (*bteA, bopB, bopD, bopN,* and *bsp22),* iron acquisition system loci (alcaligin, *alcABCDERS-fauA;* enterobactin, *bfeRAB;* and heme, *hurIR-bhuRSTUV*)*,* flagellin (*flaA*)*,* and the *Bordetella* virulence gene regulatory locus (*bvgASR*)*.*

2. The live attenuated *Bordetella bronchiseptica* HT200 strain as claimed in claim 1, wherein said strain showed an indistinctive or cryptic expression of the virulence factors - FHA (filamentous hemagglutinin) and FIM3 (serotype 3 fimbriae) but no expression of PTX-S1 (pertussis toxin subunit 1) and FIM2 (serotype 2 fimbriae).

3. The live attenuated *Bordetella bronchiseptica* HT200 strain as claimed in claim 1, wherein the sequence divergence amounts to < 98.5 % nucleotide sequence identity.

4. The live attenuated *Bordetella bronchiseptica* HT200 strain as claimed in claim 1, wherein the heterologous antigen comprises at least one epitope of a protein expressed by a pathogen responsible for an infection of the respiratory tract.

5. The live attenuated *Bordetella bronchiseptica* HT200 strain as claimed in claim 1, wherein said strain expresses hemagglutination activity, able to colonize in the respiratory organs inducing protective antibody response against infection of virulent *Bordetella bronchiseptica* RB50 strain.

6. A method for enhancing the immune response toward a microbial pathogen in a subject, said method comprising administering to a subject the live attenuated *Bordetella bronchiseptica* HT200 strain comprising a mutated pertussis toxin (p*tx)* gene due to mutation in the promoter region of said gene, deleted dermonecrotic toxin (*dnt*) gene, deleted gene for lipopolysaccharide O antigen, *ptp* operon replacing *cya* operon responsible for the secretion of adenylate cyclase hemolysin (*act*), with sequence divergence at other known *Bordetella* virulence genes and loci including the pertussis toxin production locus (*ptxABCDE*), filamentous hemagglutinin and fimbriae biogenesis loci (*fhaB-fimABCD-fhaC*), fimbrial major subunit genes (*fimN, fimX, fim2,* and *fim3*), other filamentous hemagglutinins-like genes (*fhaL,* and *fhaS*)*,* tracheal colonization factor (*tcfA*)*,* autotransporter pertactin gene (*prn*)*, Bordetella* resistance to killing genes *(brkAB),* type three secretion system (T3SS) and regulatory loci *(bsc-btr),* T3SS effector genes *(bteA, bopB, bopD, bopN,* and *bsp22),* iron acquisition system loci (alcaligin, *alcABCDERS-fauA*; enterobactin, *bfeRAB;* and heme, *hurIR-bhuRSTUV*)*,* flagellin (*flaA),* and the *Bordetella* virulence gene regulatory locus *(bvgASR)..*

7. The method as claimed in claim 7, wherein said method is designed against infection of virulent *Bordetella bronchiseptica* strain.

8. A method of reducing especially a T-lymphocyte helper type 1 (Th-1)-dominated allergic reaction in a subject against infection of *Bordetella bronchiseptica* RB50 strain, *Streptococcus pneumoniae,* Respiratory Syncytial Virus (SRV), influenza virus, said method comprises colonizing the respiratory tract of the sample subject with a live attenuated *Bordetella bronchiseptica* HT200 strain, wherein the strain having altered production of functional pertussis toxin, dermonecrotic toxin, and tracheal cytotoxin while retaining the colonizing ability, wherein the colonization of the mammal elicits an anti-inflammatory response which reduces the Th1-dominated allergic reaction in said subj ect.

9. The method as claimed in claim 8, wherein live attenuated *Bordetella bronchiseptica* HT200 strain comprising a mutated pertussis toxin (*ptx*) gene due to mutation in the promoter region of said gene, deleted dermonecrotic toxin (*dnt*) gene, deleted gene for lipopolysaccharide O antigen, *ptp* operon replacing *cya* operon responsible for the secretion of adenylate cyclase hemolysin (*act*)*.*

10. The method as claimed in claim 8, wherein colonization of the mammal with the strain occurs prior to onset of the allergic reaction in the respiratory tracts of the subject.

11. A vaccine comprising live attenuated *Bordetella bronchiseptica* HT200 strain comprising essentially of a mutated pertussis toxin (*ptx*) gene due to mutation in the promoter region of said gene, deleted dermonecrotic toxin (*dnt*) gene, deleted gene for lipopolysaccharide O antigen, *ptp* operon replacing *cya* operon responsible for the secretion of adenylate cyclase hemolysin (*act*), with sequence divergence at other known *Bordetella* virulence genes and loci including the pertussis toxin production locus (*ptxABCDE*), filamentous hemagglutinin and fimbriae biogenesis loci (*fhaB-fimABCD-fhaC*)*,* fimbrial major subunit genes (*fimN, fimX, fim2,* and *fim3*), other filamentous hemagglutinins-like genes (*fhaL,* and *fhaS*), tracheal colonization factor (*tcfA*)*,* autotransporter pertactin gene (*prn*)*, Bordetella* resistance to killing genes (*brkAB*)*,* type three secretion system (T3SS) and regulatory loci (*bsc-btr*)*,* T3SS effector genes (*bteA, bopB, bopD, bopN,* and *bsp22),* iron acquisition system loci (alcaligin, *alcABCDERS-fauA;* enterobactin, *bfeRAB;* and heme, *hurIR-bhuRSTUV*)*,* flagellin (*flaA*)*,* and the *Bordetella* virulence gene regulatory locus (*bvgASR*)*.*

12. The vaccine as claimed in claim 11, wherein said strain showed an indistinctive or cryptic expression of the virulence factors - FHA (filamentous hemagglutinin) and FIM3 (serotype 3 fimbriae) but no expression of PTX-S1 (pertussis toxin subunit 1) and FIM2 (serotype 2 fimbriae).

13. The vaccine as claimed in claim 11, wherein the sequence divergence amounts to < 98.5 % nucleotide sequence identity.

14. The vaccine as claimed in claim 11, wherein the live attenuated *Bordetella bronchiseptica* HT200 strain vaccine may be administered through inhalation or through nasal route.

15. The vaccine as claimed in claim 11, wherein said strain expresses hemagglutination activity, able to colonize in the respiratory organs inducing protective antibody response against infection of virulent *Bordetella bronchiseptica* RB50 strain.
